(19)

Europäisches
Patentamt
European
Patent Office
Office européen
des brevets

(11) **EP 4 527 375 A1**

(12) **EUROPEAN PATENT APPLICATION**
published in accordance with Art. 153(4) EPC

(43) Date of publication:
**26.03.2025 Bulletin 2025/13**

(21) Application number: **23900031.8**

(22) Date of filing: **07.12.2023**

(51) International Patent Classification (IPC):
*A61K 9/08* (2006.01)      *A61K 39/395* (2006.01)
*A61K 47/40* (2006.01)      *A61P 37/00* (2006.01)

(52) Cooperative Patent Classification (CPC):
**A61K 9/08; A61K 39/395; A61K 47/40; A61P 37/00**

(86) International application number:
**PCT/CN2023/136926**

(87) International publication number:
**WO 2024/120458 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.12.2022   CN 202211564263**

(71) Applicant: **RemeGen Co., Ltd.
Shandong 264006 (CN)**

(72) Inventors:
• **XU, Qiaoyu
Beijing Middle Road, Yantai Development Zone,
Yantai District, China (Shandong) Pilot Free
Trade Zone, Yantai, Shandong 264006 (CN)**

• **BAO, Qianjing
Beijing Middle Road, Yantai Development Zone,
Yantai District, China (Shandong) Pilot Free
Trade Zone, Yantai, Shandong 264006 (CN)**
• **LI, Qi
Beijing Middle Road, Yantai Development Zone,
Yantai District, China (Shandong) Pilot Free
Trade Zone, Yantai, Shandong 264006 (CN)**
• **ZHAO, Yating
Beijing Middle Road, Yantai Development Zone,
Yantai District, China (Shandong) Pilot Free
Trade Zone, Yantai, Shandong 264006 (CN)**
• **MA, Shuonan
Beijing Middle Road, Yantai Development Zone,
Yantai District, China (Shandong) Pilot Free
Trade Zone, Yantai, Shandong 264006 (CN)**

(74) Representative: **HGF
HGF Limited
1 City Walk
Leeds LS11 9DX (GB)**

(54) **TACI-FC FUSION PROTEIN LIQUID PHARMACEUTICAL PREPARATION**

(57)      A liquid pharmaceutical preparation. The present invention particularly relates to a liquid pharmaceutical preparation of a TACI-FC fusion protein and belongs to the field of drugs against autoimmune diseases. The pharmaceutical preparation can improve the stability of the TACI-Fc fusion protein in long-term storage, keep good biological activity, improve the efficiency of drug production, and greatly increase convenience in administration.

FIG. 1

**EP 4 527 375 A1**

**Description**

**FIELD**

The present disclosure relates to a liquid pharmaceutical preparation of TACI-Fc fusion protein, and belongs to the field of anti-autoimmune disease drugs.

**BACKGROUND**

In recent years, the biopharmaceutical market has maintained a trend of rapid growth. Fusion proteins and antibody drugs have played a pivotal role in the treatment of tumors and autoimmune diseases. With the advancement of pathology research and the development of technology, these therapies have brought benefits to patients. However, they are also susceptible to physical changes, such as aggregation, denaturation, and precipitation, and chemical changes, such as isomerization, deamidation, and oxidation. These changes can impact the safety and efficacy of the products. It is therefore necessary to ensure the stability of preparations in order to guarantee that fusion proteins and antibody drugs retain the requisite biological activity for treatment prior to administration to patients. Between 1986 and February 2021, 126 commercially available antibodies were approved worldwide, comprising 10 antibody-drug conjugates, 16 biosimilars and 3 fusion proteins. A total of 136 products for the above commercially available antibodies have been approved for commercial use, including 36 lyophilized powders and 100 liquid formulations. (Reference 1: Robert G. Strickley, et al., A review of formulations of commercially available antibodies. Journal of Pharmaceutical Sciences. 2021(110), 2590-2608.)

Although lyophilized antibody drug preparations have the advantages of long-term storage and stable activity during transportation, the lyophilization process often becomes a bottleneck step in improving drug production speed, and greatly increases the cost of drug manufacturing. In addition, lyophilized antibody drug preparations must be re-dissolved before use. In order to ensure the re-dissolution effect, the re-dissolution time is often prolonged to minimize aggregation or structural changes in the active ingredients after re-dissolution. Consequently, patients must remain in the hospital for a longer period, which places additional pressure on the hospital. Therefore, the development of stable liquid formulations is becoming an increasingly attractive option for antibody drug preparations.

Nevertheless, in comparison to lyophilized preparations, the development of antibody liquid formulations that can be stored and transported stably for an extended period remains a significant challenge for the industry. The storage of antibody liquid formulations may result in the loss of biological activity due to chemical and physical instability. A variety of chemical factors, including deamidation, racemization, hydrolysis, oxidation, $\beta$ elimination, and disulfide bond exchange, as well as physical factors such as antibody denaturation, aggregation, precipitation, and adsorption, can contribute to the instability of active ingredients.

Furthermore, the development of highly concentrated liquid formulations of antibody drugs for subcutaneous or intramuscular administration presents an additional challenge due to the potential for viscosity and protein aggregation to be significantly elevated at high concentrations. Aggregates may contain protein degraded products and can result in a range of adverse effects, including the induction of undesired immune responses.

Antibody-like structural fusion proteins or antibody preparations exhibit unique characteristics when compared to general macromolecular preparations. On the one hand, the fragile stability and structural complexity of monoclonal antibody drugs present significant challenges in terms of manufacturing and storage. The heterogeneous structure of antibodies, particularly the complementary determining region (CDR) and Fc glycosylation, necessitates a case-by-case approach to the development of formulations for different monoclonal antibodies. The development of antibody preparations presents a number of challenges, including those related to antibody conformation, colloid or chemical structure. These challenges may include oxidation, isomerization, deamidation, aggregation, denaturation, and cleavage. The three-dimensional structure of antibodies may undergo alteration, particularly in the hypervariable region (HVR), when exposed to disparate temperature and humidity, pH, and stress conditions. Undesirable products may show reduced activity, and more importantly, increased immunogenicity can endanger patients. It is therefore of paramount importance to select the most appropriate excipients in order to protect the antibodies. (Reference 2: Monoclonal antibodies: formulations of marketed products and recent advances in novel delivery system, Yanan Cui et al., Drug Development and Industrial Pharmacy, Vol. 43, No. 4, pp. 519-530, 2017). On the other hand, fusion protein products are prone to aggregation and have poor stability. The adverse immune reactions or interference with the purification process caused by them have always been a difficult problem to be solved in this field. Moreover, the factors affecting the aggregation of fusion proteins are also relatively complex, and can be divided into external factors and internal factors. The external factors mainly encompass temperature, physical pressure and solvent factors (pH, ionic strength, concentration, metal ions, etc.). The internal factors mainly include the structural characteristics of fusion proteins, sensitive residues, unpaired cysteine, etc. These factors will affect the aggregation of fusion proteins, thereby affecting their stability and shelf life. Therefore, the selection of excipients for fusion proteins requires a lot of experimental exploration (Reference 3: Production Challenges for Complex Biologics: Fusion Proteins, Stefan R. Schmidt, American Pharmaceutical Review, pp. 1-5, 2017).

Telitacicept is a first-in-class recombinant TACI-Fc fusion protein targeting B cell-related autoimmune diseases. It targets and neutralizes two key cell signaling molecules, BLyS and APRIL, in the B cell pathway. It is an antibody-like structural fusion protein composed of a truncated TACI and a sequence-optimized immunoglobulin Fc that reduces the ADCC and CDC effects for the treatment of human autoimmune system diseases, and exhibits excellent biological activity and safety. It has been approved by the China National Medical Products Administration for the treatment of systemic lupus erythematosus. Telitacicept for injection is a lyophilized preparation. For one thing, this dosage form greatly limits the production speed of the drug. For another, it significantly increases the time for patients to seek medical treatment and increases the burden on the hospital. Therefore, there is an urgent need for the development of liquid formulations.

## SUMMARY

In order to solve the above problems, the present disclosure provides a liquid pharmaceutical preparation of transmembrane activator and calcium modulator and cyclophilin ligand interactor (TACI)-Fc fusion protein, which can effectively improve the long-term stability of TACI-Fc fusion protein, maintain its good biological activity, effectively enhance the production speed of drugs, reduce drug production costs, and significantly improve patient convenience, thereby better meeting clinical drug needs.

The liquid formulation of TACI-Fc fusion protein provided by the present disclosure, has excellent stability and can be stably stored for more than 18 months under preservative-free sterile conditions at 4°C. The present disclosure also surprisingly found that the developed specific preparation composition can also meet the above requirements without using a surfactant in the preparation composition.

Specifically, the present disclosure provides a TACI-Fc fusion protein liquid formulation, which comprises a TACI-Fc fusion protein, a protective agent, a buffer and a pH regulator, wherein the TACI-Fc fusion protein comprises: (i) a TACI extracellular region or a fragment thereof binding to a B lymphocyte stimulator (Blys) and/or a proliferation-inducing ligand (APRIL), and (ii) a fragment of human immunoglobulin constant region; the protective agent is hydroxypropyl-β-cyclodextrin; the buffer is succinic acid; and the liquid formulation has a pH in the range of 5.0-5.2.

Further, the TACI-Fc fusion protein has a concentration in the range of 40 mg/ml-240 mg/ml.

Furthermore, the TACI-Fc fusion protein has a concentration of about 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/mL, 230 mg/ml, or 240 mg/ml.

Further, the content of the above fusion protein is detected by ultraviolet-visible spectroscopy. Based on the maximum UV absorption of protein at 280 nm, the absorbance value of the Telitacicept samples at this wavelength is measured. After correcting the absorbance at 320 nm, the absorbance value at 280 nm is proportional to the protein concentration. The protein concentration is calculated according to the Beer-Lambert law to determine the protein content. The calculation formula of the protein content is as follows:

$$\text{Protein content (mg/ml)} = (\text{A280 or A280 (corrected)}) / \varepsilon * \text{Sample dilution factor}$$

wherein, $\varepsilon$ is the extinction coefficient of Telitacicept, in the unit of $(\text{mg/ml})^{-1} \cdot \text{cm}^{-1}$;

A280 is the average absorbance of the sample solution at 280 nm;

A280 (corrected) is the corrected average absorbance of the sample solution at 280 nm.

Further, the protective agent has a concentration in the range of 100 mmol/L-200 mmol/L.

Furthermore, the protective agent has a concentration of about 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, or 200 mmol/L.

Further, the buffer has a concentration in the range of 5 mmol/L-15 mmol/L.

Furthermore, the buffer has a concentration of about 5 mmol/L, 6 mmol/L, 7 mmol/L, 8 mmol/L, 9 mmol/L, 10 mmol/L, 11 mmol/L, 12 mmol/L, 13 mmol/L, 14 mmol/L, or 15 mmol/L.

Further, the pH regulator is an alkaline inorganic salt, preferably sodium hydroxide.

Further, the liquid formulation has a pH of about 5.0, 5.1 or 5.2.

Preferably, the TACI-Fc fusion protein has an amino acid sequence set forth in SEQ ID NO: 1; further preferably, it is Telitacicept.

## SEQ ID NO: 1

```
SRVDQEERFP  QGLWTGVAMR  SCPEEQYWDP  LLGTCMSCKT  ICNHQSQRTC   50
AAFCRSLSCR  KEQGKFYDHL  LRDCISCASI  CGQHPKQCAY  FCENKLRSPV  100
NLPPELDKTH  TCPPCPAPEA  EGAPSVFLFP  PKPKDTLMIS  RTPEVTCVVV  150
DVSHEDPEVK  FNWYVDGVEV  HNAKTKPREE  QYNSTYRVVS  VLTVLHQDWL  200
NGKEYKCKVS  NKALPSSIEK  TISKAKGQPR  EPQVYTLPPS  RDELTKNQVS  250
LTCLVKGFYP  SDIAVEWESN  GQPENNYKTT  PPVLDSDGSF  FLYSKLTVDK  300
SRWQQGNVFS  CSVMHEALHN  HYTQKSLSLS  PGK                     333
```

Further, any one of the above-mentioned liquid formulation of TACI-Fc fusion protein comprises about 10 mmol/L of succinic acid, about 150 mmol/L of hydroxypropyl-β-cyclodextrin, and about 80 mg/mL of the TACI-Fc fusion protein, and the liquid formulation has a pH of about 5.1.

Furthermore, any one of the above-mentioned liquid formulation of TACI-Fc fusion protein comprises 10 mmol/L of succinic acid, 150 mmol/L of hydroxypropyl-β-cyclodextrin, and 80 mg/mL of the TACI-Fc fusion protein, and the liquid formulation has a pH of about 5.1.

Further, each milliliter of any one of the above-mentioned liquid formulation of TACI-Fc fusion protein comprises about 80 mg of the TACI-Fc fusion protein, about 1.18 mg of succinic acid, and about 209.85 mg of hydroxypropyl-β-cyclodextrin, and the liquid formulation has a pH of about 5.1.

Furthermore, each milliliter of any one of the above-mentioned liquid formulation of TACI-Fc fusion protein comprises 80 mg of the TACI-Fc fusion protein, 1.18 mg of succinic acid, and 209.85 mg of hydroxypropyl-β-cyclodextrin, and the liquid formulation has a pH of 5.1.

The present disclosure further relates to use of any one of the above-mentioned liquid formulation of TACI-Fc fusion protein in the manufacture of a medicament for preventing, alleviating and/or treating an autoimmune disease.

The present disclosure further relates to any one of the above-mentioned liquid formulation of TACI-Fc fusion protein for use in preventing, alleviating and/or treating an autoimmune disease.

The present disclosure further relates to a method for preventing, alleviating and/or treating an autoimmune disease, comprising administering to a subject in need thereof any one of the above-mentioned liquid formulation of TACI-Fc fusion protein.

The present disclosure further relates to a packaged pharmaceutical product, comprising any one of the above-mentioned liquid formulation of TACI-Fc fusion protein.

The present disclosure further relates to a prefilled syringe, comprising any one of the above-mentioned liquid formulation of TACI-Fc fusion protein. The prefilled syringe is further preferably a prefilled automatic syringe.

## BRIEF DESCRIPTION OF DRAWINGS

FIG. 1 shows the main effect for pH fitted by MODDE software;
FIG. 2 shows the main effect for arginine concentration fitted by MODDE software;
FIG. 3 shows the main effect for sucrose concentration fitted by MODDE software;
FIG. 4 shows the main effect for protein concentration fitted by MODDE software;
FIG. 5 shows the contour map fitted by MODDE software, from which it can be seen that it is the optimal prescription in the DOE experimental design when Arg is absent, the sucrose concentration is within 270 mmol/L-300 mmol/L, the pH is 4.95-5.1, and the protein concentration is 80 mg/mL.

## DETAILED DESCRIPTION

Unless otherwise defined, all scientific and technical terms used herein have the same meaning as understood by those of ordinary skill in the art. For definitions and terms in the art, those skilled can make a reference to Current Protocols in Molecular Biology (Ausubel).

The three-letter codes and one-letter codes of amino acids used in the present disclosure are as described in J.biol.chem, 243, p3558 (1968).

The term "TACI" in the present disclosure refers to transmembrane activator and CAML interactor, which is a member of the tumor necrosis factor receptor superfamily. The term "BLys" in the present disclosure refers to B lymphocyte stimulator, which is a member of the TNF ligand superfamily existing in two forms of membrane-bound and soluble forms, specifically expressed on the surface of bone marrow cells, and selectively stimulates the proliferation of B lymphocytes and the production of immunoglobulin. The term "APRIL" (a proliferation-inducing ligand) in the present disclosure refers to a tumor necrosis factor (TNF) analogue, which can stimulate the proliferation of primitive B cells and T cells in the body, and promote the accumulation of B cells. APRIL can specifically bind to TACI and BCMA, and the binding can prevent APRIL from binding to B cells and thus inhibit the proliferative response of primitive B cells stimulated by APRIL. Moreover, APRIL can compete with BLys for binding to receptors (BCMA and TACI).

The term "TACI-Fc fusion protein" involved in the present disclosure refers to transmembrane activator, calcium regulator and cyclophilin ligand interactor (TACI)-immunoglobulin fusion protein (i.e., TACI-Fc fusion protein). The TACI-immunoglobulin fusion protein provided by the present disclosure comprises: (i) a TACI extracellular region or a fragment thereof binding to Blys and/or APRIL; and (ii) a fragment of human immunoglobulin constant region.

For the term "TACI extracellular region or a fragment thereof binding to Blys and/or APRIL", reference can be made to the TACI extracellular domain and the specific fragment of TACI extracellular domain capable of interacting with TACI ligands disclosed in US Patent Nos. 5,969,102, 6,316,222 and 6,500,428 and US Patent Application Nos. 09/569,245 and 09/627,206, the contents of which are incorporated herein by reference, or reference can be made to the fragment of amino acids 13-118 of TACI extracellular domain disclosed in Chinese Patent Publication No. CN101323643A.

In the term "fragment of human immunoglobulin constant region", the immunoglobulin is preferably IgG1, which may comprise a heavy chain constant region, for example, a heavy chain constant region of human. The preferred "fragment of human immunoglobulin constant region" of the present disclosure is an amino acid fragment comprising a portion of the hinge region domain, a CH2 domain and a CH3 domain.

The term "treatment" involved in the present disclosure is related to a given disease or condition, including but not limited to: inhibiting the disease or condition, such as preventing the development of the disease or condition; alleviating the disease or condition, such as causing the regress of the disease or condition; or ameliorating the symptoms caused by the disease or condition, such as alleviating, preventing or treating the symptoms of the disease or condition.

The term "Telitacicept" (or "Tai'ai", which can be used interchangeably in the present disclosure) involved in the present disclosure is a TACI-Fc fusion protein, whose amino acid sequence is set forth in SEQ ID NO: 1, or as shown in https://extranet.who.int/soinn/mod/page/view.php?id=137&inn_n=10932, and its preparation can refer to the disclosure in Chinese Patent Application No. CN 101323643A or CN 113613675A.

The embodiments of the present disclosure will be described in detail below in conjunction with examples. However, those skilled in the art will understand that the following examples are only intended to illustrate the present disclosure, and should not be construed as limiting the scope of the present disclosure.

Example 1: Formulation Development

After screening and comparing a variety of excipients, this example focused on studying the effects of different concentrations of amino acids (such as arginine hydrochloride), surfactants (such as polysorbate 20 (Tween-20)), protective agents (such as sucrose) and different pH on the stability of TACI-Fc protein (amino acid sequence of SEQ ID NO: 1), with histidine used as a buffer (containing hydrochloric acid to adjust pH). The MODDE software CCF model was used to perform a 5-factor 3-level experimental design.

TACI-Fc protein (SEQ ID NO: 1) solution was dialyzed and concentrated. The mother liquor was added, adjusted to a certain volume, filtered and packaged. The resulting formula information is shown in Table 1. The prepared samples were stored at 25°C for 5 weeks (up to one month). The SEC-purity was detected at time points of 3d (i.e., day 3, the same below), 7d, 2 weeks (i.e., 2W, the same below) and 5 weeks, and the 5-week research results were analyzed using MODDE software.

Table 1 Formula Information

| No. | Histidine-Histidine hydrochloride (mmol/L) | Arginine hydrochloride (mmol/L) | Sucrose (mmol/L) | Polysorbate 20 (%) | pH | Protein concentration (mg/mL) |
|---|---|---|---|---|---|---|
| 1 | 20 | 0 | 300 | 0 | 4.8 | 80 |
| 2 | 20 | 0 | 0 | 0 | 4.8 | 160 |
| 3 | 20 | 0 | 0 | 0 | 5.2 | 80 |
| 4 | 20 | 0 | 300 | 0 | 5.2 | 160 |
| 5 | 20 | 300 | 0 | 0 | 4.8 | 80 |
| 6 | 20 | 300 | 300 | 0 | 4.8 | 160 |
| 7 | 20 | 300 | 300 | 0 | 5.2 | 80 |

(continued)

| No. | Histidine-Histidine hydrochloride ( mmol/L) | Arginine hydrochloride (mmol/L) | Sucrose (mmol/L) | Polysorbate 20 (%) | pH | Protein concentration (mg/mL) |
|---|---|---|---|---|---|---|
| 8 | 20 | 300 | 0 | 0 | 5.2 | 160 |
| 9 | 20 | 0 | 0 | 0.04 | 4.8 | 80 |
| 10 | 20 | 0 | 300 | 0.04 | 4.8 | 160 |
| 11 | 20 | 0 | 300 | 0.04 | 5.2 | 80 |
| 12 | 20 | 0 | 0 | 0.04 | 5.2 | 160 |
| 13 | 20 | 300 | 300 | 0.04 | 4.8 | 80 |
| 14 | 20 | 300 | 0 | 0.04 | 4.8 | 160 |
| 15 | 20 | 300 | 0 | 0.04 | 5.2 | 80 |
| 16 | 20 | 300 | 300 | 0.04 | 5.2 | 160 |
| 17 | 20 | 150 | 150 | 0.02 | 5.0 | 80 |
| 18 | 20 | 150 | 150 | 0.02 | 5.0 | 160 |
| 19 | 20 | 150 | 150 | 0.02 | 4.8 | 120 |
| 20 | 20 | 150 | 150 | 0.02 | 5.2 | 120 |
| 21 | 20 | 0 | 150 | 0.02 | 5.0 | 120 |
| 22 | 20 | 300 | 150 | 0.02 | 5.0 | 120 |
| 23 | 20 | 150 | 150 | 0 | 5.0 | 120 |
| 24 | 20 | 150 | 150 | 0.04 | 5.0 | 120 |
| 25 | 20 | 150 | 0 | 0.02 | 5.0 | 120 |
| 26 | 20 | 150 | 300 | 0.02 | 5.0 | 120 |
| 27 | 20 | 150 | 150 | 0.02 | 5.0 | 120 |
| 28 | 20 | 150 | 150 | 0.02 | 5.0 | 120 |
| 29 | 20 | 150 | 150 | 0.02 | 5.0 | 120 |

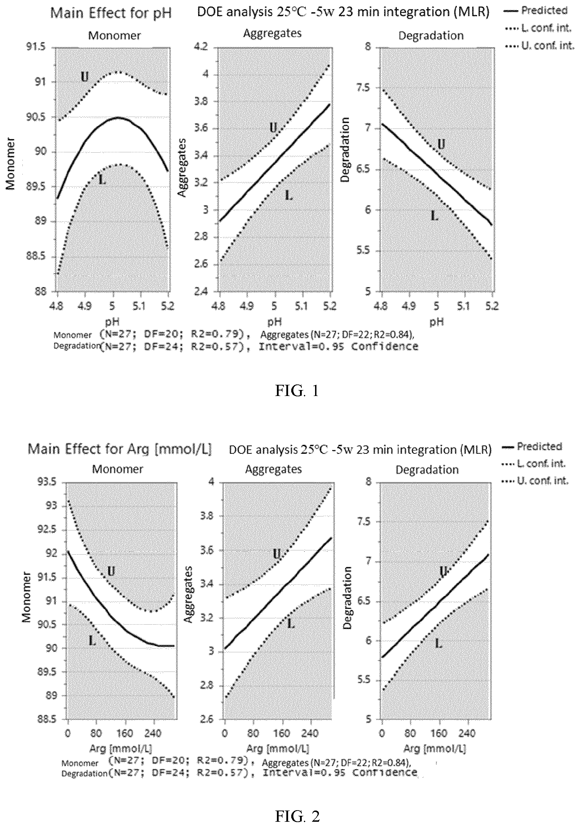

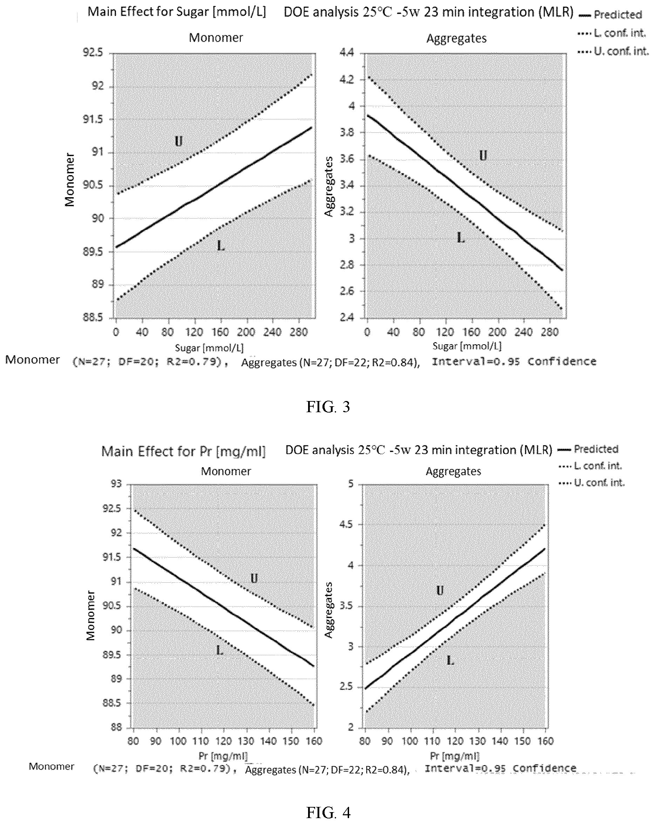

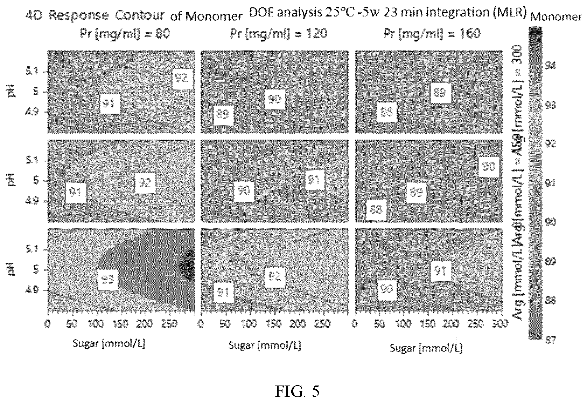

The experimental analysis results are shown in FIGs. 1, 2, 3, and 4. The results show that the effects of arginine hydrochloride concentration and sucrose concentration on protein purity are statistically significant (P<0.05). With the increase of arginine hydrochloride concentration, the aggregates increased, resulting in a decrease in protein purity. On the contrary, with the increase of sucrose concentration, the aggregates decreased, resulting in an increase in protein purity. Surprisingly, the presence of Tween-20 had no effect on the decrease of aggregates and degradation, indicating that surfactants can be omitted in the preparation. The pH value had no major effect on the change of monomer content (P>0.05), indicating that pH values in the range of 4.8-5.2 were suitable for the liquid protein preparation of Telitacicept. It can be seen from the contour map of FIG. 5 that it was a more suitable formula when arginine hydrochloride was absent, the sucrose concentration was between 270 mmol/L and 300 mmol/L, and the protein concentration was 80 mg/mL.

Example 2: Buffer system screening

This example compared the effects of three buffer systems: histidine-histidine hydrochloride buffer system, citric acid buffer system, and succinic acid buffer system, to screen for the buffer system that best matched the TACI-Fc protein preparation. The concentration of TACI-Fc protein (SEQ ID NO: 1) was selected to be 160 mg/mL, pH was 5.0, and the sucrose concentration was 290 mmol/L.

TACI-Fc protein (SEQ ID NO: 1) was dialyzed and concentrated into the formula in Table 2, adjusted to a certain volume, filtered and packaged. The prepared samples were then stored at 25°C, and the SEC- purity was detected at 3d and 9d.

Table 2 Buffer system formula information

| No. | Buffer system | Sucrose (mmol/L) | pH | Protein concentration (mg/mL) |
|---|---|---|---|---|
| 1 | 10 mmol/L Histidine-Histidine hydrochloride buffer system | 290 | 5.0 | 160 |
| 2 | 10 mmol/L Citric acid buffer system | 290 | 5.0 | 160 |
| 3 | 10 mmol/L Succinic acid buffer system | 290 | 5.0 | 160 |

Table 3 Purity (SEC-purity) (%) Results

| No. | 0h | | | 25°C-3d | | | 25°C-9d | | |
|---|---|---|---|---|---|---|---|---|---|
| | Aggregates | Monomer | Degradation | Aggregates | Monomer | Degradation | Aggregates | Monomer | Degradation |
| 1 | 2.946 | 97.054 | 0.000 | 4.136 | 95.864 | 0.000 | 4.097 | 95.903 | 0.000 |
| 2 | 2.189 | 97.811 | 0.000 | 3.596 | 96.404 | 0.000 | 4.786 | 95.214 | 0.000 |
| 3 | 1.877 | 98.123 | 0.000 | 2.865 | 97.135 | 0.000 | 3.682 | 96.318 | 0.000 |

Table 4 Results of visible foreign matter

| No. | 0h | 25°C-3d |
|---|---|---|
| 1 | Clear | Clear |
| 2 | White | White |
| 3 | Clear | Clear |

Table 5 Viscosity (mPa.s) Results

| No. | 0h | | 25°C-3d | |
|---|---|---|---|---|
| | Viscosity | Temperature | Viscosity | Temperature |
| 1 | 9.67 | 24.4 | 8.18 | 24.3 |
| 2 | 17.43 | 23.8 | 18.35 | 23.7 |
| 3 | 9.44 | 25.0 | 10.06 | 23.3 |

Table 6 Protein Concentration (mg/ml) Results

| No. | 0h | 25°C-3d |
|---|---|---|
| 1 | 149.62 | 154.06 |
| 2 | 174.07 | 152.23 |
| 3 | 151.82 | 148.32 |

Table 7 pH Changes

| No. | 0h | 25°C-3d |
|---|---|---|
| 1 | 5.117 | 5.018 |
| 2 | 4.999 | 5.128 |
| 3 | 4.997 | 4.939 |

The experimental results are shown in Tables 3 to 7. The results show that after being placed at 25°C for 9 days, the main peak purity of the histidine-histidine hydrochloride buffer system, the citric acid buffer system, and the succinic acid buffer system decreased by 1.2%, 2.6%, and 1.8%, respectively. After being placed at 25°C for 9 days, all three buffer systems had an increase in aggregates, and the increase in aggregates in the histidine-histidine hydrochloride buffer system, the citric acid buffer system, and the succinic acid buffer system were 4.1%, 4.8%, and 3.7%, respectively. Besides, from the visible foreign matter and viscosity results, it can be seen that the citric acid buffer system had worse stability than the other two groups, and exhibited whitish appearance and large viscosity, which is not suitable for the protein preparation. In addition, in the citric acid buffer system, the TACI-Fc protein was more difficult to dialysis than the other two groups, and after dialysis, the protein became a milky suspension after being placed at 4°C for a period of time, and then returned to a white, clear liquid after being placed at room temperature for a period of time. Furthermore, in the citric acid buffer system, the viscosity of the TACI-Fc protein after dialysis was relatively large compared with the other two groups, and the protein solution was more viscous. In summary, the histidine-histidine hydrochloride buffer system and the succinic acid buffer system were better than the citric acid buffer system. In addition, the study found that at pH 5.0, the buffering capacity of the succinic acid buffer system was better than that of the histidine-histidine hydrochloride buffer system.

Example 3: Protective agent screening

In Example 1, it has been found that protein stability increased with increasing sucrose concentration. The possible reason is that the hydroxyl group in sucrose acts as a proton donor, which inhibits the deprotonation of thiols to a certain extent, thereby reducing the formation rate of disulfide bond aggregates. However, high concentrations of sucrose may lead to a significant increase in the osmotic pressure of the liquid formulation, thereby affecting the isotonicity of the preparation required for subcutaneous injection, and may cause higher levels of endotoxins. After evaluating and comparing a variety of different protective agents, this example focused on the effect of hydroxypropyl-β-cyclodextrin as a protective agent on the stability of the preparation.

TACI-Fc protein (SEQ ID NO: 1) was dialyzed and concentrated into the formula in Table 8, adjusted to a certain volume, filtered and packaged. The prepared samples were then stored at 25°C and 40°C, respectively. SEC-purity was detected at 4d and 7d at 40°C and detected at 2 weeks, 3 weeks and 4 weeks at 25 °C. The capillary electrophoresis (nrCE-SDS) changes were detected at 40°C, and protein cell activity and binding activity were detected at 25°C.

Table 8 Formula information of protective agent

| No. | Succinic acid (mmol/L) | Sucrose (mmol/L) | Hydroxypropyl-β-cyclodextrin (mmol/L) | Protein concentration (mg/mL) | pH |
|---|---|---|---|---|---|
| 1 | 10 | / | 50 | 80 | 5.1 |
| 2 | 10 | / | 140 | 80 | 5.1 |
| 3 | 10 | 180 | / | 80 | 5.1 |
| 4 | 10 | 300 | / | 80 | 5.1 |

Table 9 Results of purity (SEC-purity) (%) at 40°C

| No. | 0h | | | 40°C-4d | | | 40°C-7d | | |
|---|---|---|---|---|---|---|---|---|---|
| | Aggregates | Monomer | Degradation | Aggregates | Monomer | Degradation | Aggregates | Monomer | Degradation |
| 1 | 1.6 | 98.4 | 0.0 | 5.8 | 93.7 | 0.4 | 6.8 | 91.2 | 2.0 |
| 2 | 1.5 | 98.5 | 0.0 | 3.9 | 95.8 | 0.3 | 4.7 | 93.6 | 1.7 |
| 3 | 1.9 | 98.1 | 0.0 | 6.8 | 92.8 | 0.4 | 7.9 | 91.3 | 0.8 |
| 4 | 1.8 | 98.2 | 0.0 | 5.2 | 94.2 | 0.5 | 6.3 | 92.7 | 1.1 |

Table 10 Results of purity (SEC-purity) (%) at 25°C

| No. | 0h | | | 25°C-2w | | | 25°C-3w | | | 25°C-4w | | |
|---|---|---|---|---|---|---|---|---|---|---|---|---|
| | Aggregates | Monomer | Degradation | Aggregates | Monomer | Degradation | Aggregates | Monomer | Degradation | Aggregates | Monomer | Degradation |
| 1 | 1.6 | 98.4 | 0.0 | 2.9 | 96.5 | 0.6 | 3.4 | 95.3 | 1.3 | 3.4 | 95.0 | 1.7 |
| 2 | 1.5 | 98.5 | 0.0 | 2.2 | 97.3 | 0.5 | 2.7 | 96.3 | 1 | 2.6 | 96.2 | 1.3 |
| 3 | 1.9 | 98.1 | 0.0 | 3.7 | 95.6 | 0.6 | 4.3 | 94.5 | 1.2 | 4.2 | 94.4 | 1.5 |
| 4 | 1.8 | 98.2 | 0.0 | 3.0 | 96.2 | 0.8 | 3.5 | 94.9 | 1.5 | 3.4 | 94.8 | 1.8 |

Table 11 nrCE-SDS results at 40°C

| No. | 0h | 40°C-4d | 40°C-7d |
|---|---|---|---|
| 1 | 98.2 | 93.7 | 94.1 |
| 2 | 98.2 | 95.8 | 96.1 |
| 3 | 97.9 | 92.7 | 93.1 |
| 4 | 97.9 | 94.1 | 94.2 |

Table 12 Cell viability results at 25°C

| No. | 0h | 25°C-2w |
|---|---|---|
| 1 | 87% | 101% |
| 2 | 66% | 88% |
| 3 | 94% | 96% |

(continued)

| No. | 0h | 25°C-2w |
|---|---|---|
| 4 | 103% | 103% |

Table 13 Binding activity results at 25°C

| No. | 0h | 25°C-2w |
|---|---|---|
| 1 | 82.4% | 98.7% |
| 2 | 88.2% | 77.7% |
| 3 | 87.8% | 99.2% |
| 4 | 83.8% | 108.4% |

The experimental results are shown in Tables 9-13. The SEC results at 40°C and 25°C (see Tables 9-10) show that higher hydroxypropyl-β-cyclodextrin concentration led to better TACI-Fc protein stability, higher sucrose concentration led to better protein stability, and the protective effect of hydroxypropyl-β-cyclodextrin on TACI-Fc protein was better than that of sucrose on the protein. The nrCE-SDS results (see Table 11) show that higher hydroxypropyl-β-cyclodextrin concentration led to better protein stability, higher sucrose concentration led to better protein stability, and the protective effect of hydroxypropyl-β-cyclodextrin on the protein was better than that of sucrose on the protein, which was consistent with the results in Tables 9-10. The results of cell activity at 25°C (see Table 12) show no decreasing trend in the cell activity of the two. The results of binding activity at 25°C (see Table 13) show no decreasing trend in the binding activity of the two. From the above results, it can be seen that the presence of sucrose or hydroxypropyl-β-cyclodextrin reduced the formation of protein aggregates and the degraded, and the protective effect of hydroxypropyl-β-cyclodextrin on TACI-Fc protein was better than sucrose.

Example 4: pH confirmation experiment

This example focused on the effect of different pH values on the stability of TACI-Fc protein based on a hydroxypropyl-β-cyclodextrin concentration of 140 mmol/L and a succinic acid buffer system of 10 mmol/L.
TACI-Fc protein (SEQ ID NO: 1) was dialyzed into 10 mmol/L succinic acid, concentrated to 40 mg/mL, and adjusted to a certain volume. Then the mother liquor was added and concentrated to the target concentration. The final formula information is shown in Table 14. Then the prepared sample was stored at 40°C. The SEC-purity was detected at the time points of 2d and 7d.

Table 14 Formula information for pH confirmation

| No. | Succinic acid (mmol/L) | Hydroxypropyl-β-cyclodextrin (mmol/L) | Protein concentration (mg/mL) | pH |
|---|---|---|---|---|
| 1 | 10 | 140 | 80 | 5.0 |
| 2 | 10 | 140 | 80 | 5.2 |
| 3 | 10 | 140 | 80 | 5.5 |

Table 15 Results of purity (SEC-purity) (%)at 40°C

| No. | 0h | | | 40°C-2d | | | 40°C-7d | | |
|---|---|---|---|---|---|---|---|---|---|
| | Aggregates | Monomer | Degradation | Aggregates | Monomer | Degradation | Aggregates | Monomer | Degradation |
| 1 | 1.5 | 98.5 | 0.0 | 2.7 | 97.0 | 0.2 | 4.8 | 93.2 | 2.0 |
| 2 | 1.5 | 98.5 | 0.0 | 3.6 | 96.2 | 0.2 | 6.2 | 92.3 | 1.6 |
| 3 | 1.6 | 98.5 | 0.0 | 4.8 | 95.1 | 0.1 | 7.9 | 91.6 | 0.5 |

The experimental results are shown in Table 15. The results show that as the pH increased, the aggregates tended to increase and the degraded tended to decrease, but the increase in the aggregates was greater than the decrease in the degraded, which made the monomer tend to decrease. In order to balance the aggregates and degraded degree, the pH was determined to be 5.0-5.2.

Example 5: Hydroxypropyl-β-cyclodextrin concentration confirmation experiment

This example focused on the effect of 50 mmol/L, 100 mmol/L and 140 mmol/L hydroxypropyl-β-cyclodextrin on protein stability at pH 5.1 with 10 mmol/L succinic acid and 80 mmol/L TACI-Fc (SEQ ID NO: 1).

TACI-Fc (SEQ ID NO: 1) was dialyzed and concentrated into the formula in Table 16, adjusted to a certain volume, filtered and packaged. The prepared samples were then stored at 40°C. The SEC-purity, nrCE-SDS and osmotic pressure were detected at time points of 3d and 7d.

Table 16 Formula information

| No. | Succinic acid (mmol/L) | Hydroxypropyl-β-cyclodextrin (mmol/L) | Protein concentration (mg/mL) | pH |
|---|---|---|---|---|
| 1 | 10 | 140 | 80 | 5.1 |
| 2 | 10 | 100 | 80 | 5.1 |
| 3 | 10 | 50 | 80 | 5.1 |

Table 17 Results of purity (SEC-purity) (%) at 40°C

| No. | 0h | | | 40°C-3d | | | 40°C-7d | | |
|---|---|---|---|---|---|---|---|---|---|
| | Aggregates | Monomer | Degradation | Aggregates | Monomer | Degradation | Aggregates | Monomer | Degradation |
| 1 | 1.6 | 98.4 | 0.0 | 3.9 | 95.8 | 0.2 | 4.7 | 93.7 | 1.6 |
| 2 | 1.6 | 98.4 | 0.0 | 4.2 | 95.5 | 0.3 | 5.1 | 92.8 | 2.1 |
| 3 | 1.6 | 98.4 | 0.0 | 4.3 | 95.3 | 0.4 | 5.1 | 92.7 | 2.1 |

Table 18 nrCE-SDS results

| No. | 0h | 40°C-3d | 40°C-7d |
|---|---|---|---|
| 1 | 98.4% | 96.7% | 95.7% |
| 2 | 98.4% | 96.6% | 95.4% |
| 3 | 98.4% | 96.4% | 95.2% |

Table 19 Osmotic pressure (mOsm/kg) results

| No. | 0h | 40°C-3d | 40°C-7d |
|---|---|---|---|
| 1 | 267 | 277 | 276 |
| 2 | 238 | 239 | 236 |
| 3 | 131 | 130 | 137 |

The experimental results are shown in Tables 17 to 19, wherein the results in Tables 17 and 18 show that as the concentration of hydroxypropyl-β-cyclodextrin decreased, the purity decreased successively. The results in Table 19 show that the isotonic effect can only be initially achieved when the concentration of hydroxypropyl-β-cyclodextrin was 140 mmol/L. The concentration of hydroxypropyl-β-cyclodextrin will be increased subsequently to obtain a more reasonable solution osmotic pressure. The concentration of hydroxypropyl-β-cyclodextrin was further set at 150 mmol/L and confirmed by an osmotic pressure experiment.

Example 6: Osmotic pressure confirmation

The experiment was carried out in two parallel groups A and B as follows. The specific formula information is shown in Table 20.

Table 20 Formula information

| Group | No. | Succinic acid (mmol/L) | Target pH | Protein concentration (mg/mL) | Hydroxypropyl-β-cyclodextrin (mmol/L) | Preparation method |
|---|---|---|---|---|---|---|
| A | 1 | 10 | 5.1 | 80 | 150 | Adding mother liquor |
| | 2 | 10 | 5.1 | 120 | 150 | |
| B | 3 | 10 | 5.1 | 80 | 150 | Adding mother liquor |
| | 4 | 10 | 5.1 | 120 | 150 | |

Table 21 Osmotic pressure (mOsm/kg) and pH Results

| Group | No. | Osmotic pressure, concentration and pH of protein after adding mother liquor | | | |
|---|---|---|---|---|---|
| | | Osmotic pressure (before filtration) | Osmotic pressure (after filtration) | Concentration | pH |
| A | 1 | 333 | / | 80.12 | 5.107 |
| | 2 | 392 | 397 | 122.22 | 5.147 |
| B | 3 | 328 | / | 81.07 | 5.132 |
| | 4 | 406 | / | 114.37 | 5.192 |

The experimental results are shown in Table 21, which show that when the concentration of TACI-Fc (SEQ ID NO: 1) was 80 mg/mL, the osmotic pressure of both groups A and B was about 330; when the concentration of TACI-Fc (SEQ ID NO: 1) was 120 mg/mL, the osmotic pressure of both groups A and B was about 400. The osmotic pressure measured before and after filtration was not much different, indicating that filtration had no effect on the osmotic pressure, and the pH was all within the range of 5.1±0.1. In summary, the composition of the preparation was preliminarily determined to be: 10 mmol/L succinic acid, 150 mmol/L hydroxypropyl-β-cyclodextrin, protein concentration of 80 mg/mL, and pH adjusted to 5.1±0.1 with sodium hydroxide.

Example 7: Long-term stability test

This example focused on the long-term and accelerated stability of the finalized formula stock solution packaged into prefilled syringes.
TACI-Fc (SEQ ID NO: 1) was dialyzed into the following formulas, then concentrated, adjusted to a certain volume, filtered and packaged. The specific formula information is shown in Table 22. According to the temperature and time parameters in Table 23, the long-term stability of the samples under different temperature conditions was investigated.

Table 22 Formula information

| No. | Succinic acid (mmol/L) | Hydroxypropyl-β-cyclodextrin (mmol/L) | Protein concentration (mg/mL) | pH |
|---|---|---|---|---|
| 1 | 10 | 150 | 80 | 5.1 |

Table 23 Investigation time points for long-term stability

| Conditions | Time points (m) |
|---|---|
| -20°C | 0h, 3, 6, 9, 12, 18, 24 |
| 4°C | 0h, 3, 6, 9, 12, 18, 24 |
| 25°C | 0h, 1, 2, 3, 6 |

According to SEC-purity data, after TACI-Fc (SEQ ID NO: 1) was placed at -20 to 4°C for 24 months, the aggregates increased from 1.3% to 2.88%, and the purity decreased from 98.6% to 94.7%, both of which were within the quality standard range. In addition, no increase in particulate matter (visible foreign matter and insoluble particles) was observed, and all results were within the acceptable standard range. Other product quality attributes including peptide map, appearance, osmotic pressure, pH, CE-SDS (r/nr), RP-HPLC, activity, protein content, bacterial endotoxin and container closure integrity (CCI) data were almost unchanged, and all results were within the acceptable standard range. After strict long-term stability evaluation, the results show that after the preparation of the present disclosure was filled in a prefilled automatic syringe (PFS) and stored at -20 to 4°C for a long time, it exhibited good stability, which met the requirements of the stability quality standard. Moreover, even after the preparation of the present disclosure was stored at 25°C for 6 months, despite the slightly increased aggregates detected by SEC-purity, all test results were within the quality standard range.

The above is a detailed description of the spirit of the present disclosure through the preferred embodiments of the present disclosure. Those skilled in the art understand that any modification, equivalent changes and modifications made to the above embodiments based on the technical essence of the present disclosure fall within the protection scope of the present disclosure.

**Claims**

1.  A liquid formulation of transmembrane activator and calcium modulator and cyclophilin ligand interactor (TACI)-Fc fusion protein, comprising a TACI-Fc fusion protein, a protective agent, a buffer and a pH regulator, wherein the TACI-Fc fusion protein comprises: (i) a TACI extracellular region or a fragment thereof binding to a B lymphocyte stimulator (Blys) and/or a proliferation-inducing ligand (APRIL), and (ii) a fragment of human immunoglobulin constant region; wherein the protective agent is hydroxypropyl-β-cyclodextrin; the buffer is succinic acid; and the liquid formulation has a pH of 5.0-5.2.

2.  The liquid formulation of TACI-Fc fusion protein according to claim 1, wherein the TACI-Fc fusion protein has a concentration in the range of 40 mg/ml-240 mg/ml.

3.  The liquid formulation of TACI-Fc fusion protein according to claim 2, wherein the TACI-Fc fusion protein has a concentration of about 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/mL, 230 mg/ml, or 240 mg/ml.

4.  The liquid formulation of TACI-Fc fusion protein according to claim 2 or 3, wherein the protective agent has a concentration in the range of 100 mmol/L-200 mmol/L.

5.  The liquid formulation of TACI-Fc fusion protein according to claim 4, wherein the protective agent has a concentration of about 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, or 200 mmol/L.

6.  The liquid formulation of TACI-Fc fusion protein according to claim 4 or 5, wherein the buffer has a concentration in the range of 5 mmol/L-15 mmol/L.

7.  The liquid formulation of TACI-Fc fusion protein according to claim 6, wherein the buffer has a concentration of about 5 mmol/L, 6 mmol/L, 7 mmol/L, 8 mmol/L, 9 mmol/L, 10 mmol/L, 11 mmol/L, 12 mmol/L, 13 mmol/L, 14 mmol/L, or 15 mmol/L.

8.  The liquid formulation of TACI-Fc fusion protein according to claim 6 or 7, wherein the pH regulator is an alkaline inorganic salt.

9.  The liquid formulation of TACI-Fc fusion protein according to claim 8, wherein the pH regulator is sodium hydroxide.

10.  The liquid formulation of TACI-Fc fusion protein according to claim 8 or 9, wherein the liquid formulation has a pH of about 5.0, 5.1 or 5.2.

11.  The liquid formulation of TACI-Fc fusion protein according to claim 10, wherein the TACI-Fc fusion protein has an amino acid sequence set forth in SEQ ID NO: 1.

12.  The liquid formulation of TACI-Fc fusion protein according to claim 11, wherein the TACI-Fc fusion protein is Telitacicept.

13. The liquid formulation of TACI-Fc fusion protein according to claim 11 or 12, wherein the liquid formulation comprises about 10 mmol/L of succinic acid, about 150 mmol/L of hydroxypropyl-β-cyclodextrin, and about 80 mg/mL of the TACI-Fc fusion protein, and the liquid formulation has a pH of about 5.1.

14. The liquid formulation of TACI-Fc fusion protein according to claim 11 or 12, wherein each milliliter of the liquid formulation comprises about 80 mg of the TACI-Fc fusion protein, about 1.18 mg of succinic acid, about 209.85 mg of hydroxypropyl-β-cyclodextrin, and the liquid formulation has a pH of about 5.1.

15. Use of the liquid formulation of TACI-Fc fusion protein according to any one of claims 1 to 14 in the manufacture of a medicament for preventing, alleviating and/or treating an autoimmune disease.

16. The liquid formulation of TACI-Fc fusion protein according to any one of claims 1 to 14 for use in preventing, alleviating and/or treating an autoimmune disease.

17. A method for preventing, alleviating and/or treating an autoimmune disease, comprising administering to a subject in need thereof the liquid formulation of TACI-Fc fusion protein according to any one of claims 1 to 14.

18. A packaged pharmaceutical product comprising the liquid formulation of TACI-Fc fusion protein according to any one of claims 1 to 14.

19. A prefilled syringe comprising the liquid formulation of TACI-Fc fusion protein according to any one of claims 1 to 14.

20. The syringe according to claim 19, wherein the syringe is a prefilled automatic syringe.

**Amended claims under Art. 19.1 PCT**

1. A liquid formulation of transmembrane activator and calcium modulator and cyclophilin ligand interactor (TACI)-Fc fusion protein, comprising a TACI-Fc fusion protein and a protective agent, wherein the TACI-Fc fusion protein comprises: (i) a TACI extracellular region or a fragment thereof binding to a B lymphocyte stimulator (Blys) and/or a proliferation-inducing ligand (APRIL), and (ii) a fragment of human immunoglobulin constant region; wherein the protective agent is hydroxypropyl-β-cyclodextrin.

2. The liquid formulation of TACI-Fc fusion protein according to claim 1, wherein the TACI-Fc fusion protein has a concentration in the range of 40 mg/ml-240 mg/ml.

3. The liquid formulation of TACI-Fc fusion protein according to claim 2, wherein the TACI-Fc fusion protein has a concentration of about 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/mL, 230 mg/ml, or 240 mg/ml.

4. The liquid formulation of TACI-Fc fusion protein according to claim 2 or 3, wherein the protective agent has a concentration in the range of 100 mmol/L-200 mmol/L.

5. The liquid formulation of TACI-Fc fusion protein according to claim 4, wherein the protective agent has a concentration of about 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, or 200 mmol/L.

6. The liquid formulation of TACI-Fc fusion protein according to claim 4, wherein the liquid formulation further comprises a buffer and/or a pH regulator.

7. The liquid formulation of TACI-Fc fusion protein according to claim 1 or 6, wherein the liquid formulation does not comprise any surfactant.

8. The liquid formulation of TACI-Fc fusion protein according to claim 6 or 7, wherein the buffer is succinic acid and has a concentration in the range of 5 mmol/L-15 mmol/L.

9. The liquid formulation of TACI-Fc fusion protein according to claim 8, wherein the buffer has a concentration of about 5 mmol/L, 6 mmol/L, 7 mmol/L, 8 mmol/L, 9 mmol/L, 10 mmol/L, 11 mmol/L, 12 mmol/L, 13 mmol/L, 14 mmol/L, or 15 mmol/L.

10. The liquid formulation of TACI-Fc fusion protein according to claim 6 or 8, wherein the pH regulator is an alkaline inorganic salt.

11. The liquid formulation of TACI-Fc fusion protein according to claim 10, wherein the pH regulator is sodium hydroxide.

12. The liquid formulation of TACI-Fc fusion protein according to claim 11, wherein the liquid formulation has a pH in the range of 4.8-5.2.

13. The liquid formulation of TACI-Fc fusion protein according to claim 12, wherein the liquid formulation has a pH of about 4.8, 4.9, 5.0, 5.1, or 5.2.

14. The liquid formulation of TACI-Fc fusion protein according to claim 13, wherein the TACI-Fc fusion protein has an amino acid sequence set forth in SEQ ID NO: 1.

15. The liquid formulation of TACI-Fc fusion protein according to claim 14, wherein the TACI-Fc fusion protein is Telitacicept.

16. The liquid formulation of TACI-Fc fusion protein according to claim 15, wherein the liquid formulation comprises about 10 mmol/L of succinic acid, about 150 mmol/L of hydroxypropyl-$\beta$-cyclodextrin, and about 80 mg/mL of the TACI-Fc fusion protein, and the liquid formulation has a pH of about 5.1.

17. The liquid formulation of TACI-Fc fusion protein according to claim 15, wherein each milliliter of the liquid formulation comprises about 80 mg of the TACI-Fc fusion protein, about 1.18 mg of succinic acid, about 209.85 mg of hydroxypropyl-$\beta$-cyclodextrin, and the liquid formulation has a pH of about 5.1.

18. Use of the liquid formulation of TACI-Fc fusion protein according to any one of claims 1 to 17 in the manufacture of a medicament for preventing, alleviating and/or treating an autoimmune disease.

19. The liquid formulation of TACI-Fc fusion protein according to any one of claims 1 to 17 for use in preventing, alleviating and/or treating an autoimmune disease.

20. A method for preventing, alleviating and/or treating an autoimmune disease, comprising administering to a subject in need thereof the liquid formulation of TACI-Fc fusion protein according to any one of claims 1 to 17.

21. A packaged pharmaceutical product comprising the liquid formulation of TACI-Fc fusion protein according to any one of claims 1 to 17.

22. A prefilled syringe comprising the liquid formulation of TACI-Fc fusion protein according to any one of claims 1 to 17.

23. The syringe according to claim 22, wherein the syringe is a prefilled automatic syringe.

24. Use of hydroxypropyl-$\beta$-cyclodextrin in the manufacture of a liquid formulation comprising TACI-Fc fusion protein.

25. The use according to claim 24, wherein the TACI-Fc fusion protein has an amino acid sequence set forth in SEQ ID NO: 1.

FIG. 1

FIG. 2

Main Effect for Sugar [mmol/L]    DOE analysis 25°C -5w 23 min integration (MLR) — Predicted
···· L. conf. int.
···· U. conf. int.

Monomer  (N=27; DF=20; R2=0.79),  Aggregates (N=27; DF=22; R2=0.84),  Interval=0.95 Confidence

FIG. 3

Main Effect for Pr [mg/ml]    DOE analysis 25°C -5w 23 min integration (MLR) — Predicted
···· L. conf. int.
···· U. conf. int.

Monomer  (N=27; DF=20; R2=0.79),  Aggregates (N=27; DF=22; R2=0.84),  Interval=0.95 Confidence

FIG. 4

FIG. 5

## INTERNATIONAL SEARCH REPORT

| International application No. |
| --- |
| **PCT/CN2023/136926** |

### A. CLASSIFICATION OF SUBJECT MATTER

A61K9/08(2006.01)i; A61K39/395(2006.01)i; A61K47/40(2006.01)i; A61P37/00(2006.01)i

According to International Patent Classification (IPC) or to both national classification and IPC

### B. FIELDS SEARCHED

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

CNTXT, CNABS, CAPLUS, DWPI, WPABS, WPABSC, ENTXTC, ENTXT, CJFD, CNKI, BAIDU, EMBASE, MEDLINE, NCBI, 中国专利生物序列检索系统, China Patent Biological Sequence Search System: 荣昌, TACI-Fc融合蛋白, 泰它西普, 环糊精, 琥珀酸, REMEGEN, TACI-Fc fusion protein, Telitacicept, Cyclodextrin, succinic acid, SEQ ID NO: 1

### C. DOCUMENTS CONSIDERED TO BE RELEVANT

| Category* | Citation of document, with indication, where appropriate, of the relevant passages | Relevant to claim No. |
| --- | --- | --- |
| PA | CN 116867507 A (REMEGEN BIOPHARMACEUTICAL (YANTAI) CO., LTD.) 10 October 2023 (2023-10-10)<br>entire document | 1-20 |
| A | CN 113613675 A (REMEGEN BIOPHARMACEUTICAL (YANTAI) CO., LTD.) 05 November 2021 (2021-11-05)<br>claims 1-21 | 1-20 |
| A | CN 101323643 A (RC BIOTECHNOLOGIES, LTD.) 17 December 2008 (2008-12-17)<br>entire document | 1-20 |
| A | CN 102085367 A (RC BIOTECHNOLOGIES, LTD.) 08 June 2011 (2011-06-08)<br>entire document | 1-20 |
| A | CN 102085368 A (RC BIOTECHNOLOGIES, LTD.) 08 June 2011 (2011-06-08)<br>entire document | 1-20 |
| A | CN 110522908 A (REMEGEN BIOPHARMACEUTICAL (YANTAI) CO., LTD.) 03 December 2019 (2019-12-03)<br>entire document | 1-20 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **01 February 2024** | **20 February 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/136926** |

### C.    DOCUMENTS CONSIDERED TO BE RELEVANT

| Category* | Citation of document, with indication, where appropriate, of the relevant passages | Relevant to claim No. |
| --- | --- | --- |
| A | US 2021363223 A1 (ALPINE IMMUNE SCIENCES INC.) 25 November 2021 (2021-11-25) entire document | 1-20 |

**INTERNATIONAL SEARCH REPORT**

International application No.

**PCT/CN2023/136926**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2023/136926**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **17**
   because they relate to subject matter not required to be searched by this Authority, namely:

   Claim 17 relates to "a method for preventing, alleviating and/or treating autoimmune diseases", which falls within methods for treatment of diseases, and thus said claim does not comply with PCT Rule 39.1(iv). In the present search report, a search is conducted on the basis that the designation of the subject matter is a pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

| INTERNATIONAL SEARCH REPORT | | | | | International application No. | |
|---|---|---|---|---|---|---|
| Information on patent family members | | | | | PCT/CN2023/136926 | |

| Patent document cited in search report | | | Publication date (day/month/year) | Patent family member(s) | | | Publication date (day/month/year) |
|---|---|---|---|---|---|---|---|
| CN | 116867507 | A | 10 October 2023 | WO | 2023016444 | A1 | 16 February 2023 |
| | | | | EP | 4292603 | A1 | 20 December 2023 |
| | | | | US | 2024002468 | A1 | 04 January 2024 |
| | | | | AU | 2022325359 | A1 | 18 May 2023 |
| | | | | AU | 2022325359 | A9 | 29 June 2023 |
| | | | | TW | 202333770 | A | 01 September 2023 |
| | | | | CA | 3196569 | A1 | 16 February 2023 |
| CN | 113613675 | A | 05 November 2021 | CA | 3128113 | A1 | 17 June 2021 |
| | | | | EP | 4074337 | A1 | 19 October 2022 |
| | | | | EP | 4074337 | A4 | 13 December 2023 |
| | | | | WO | 2021115321 | A1 | 17 June 2021 |
| | | | | KR | 20220054324 | A | 02 May 2022 |
| | | | | AU | 2020401160 | A1 | 19 August 2021 |
| | | | | BR | 112021025458 | A2 | 21 June 2022 |
| | | | | JP | 2022546899 | A | 10 November 2022 |
| | | | | JP | 7326500 | B2 | 15 August 2023 |
| | | | | US | 2022133633 | A1 | 05 May 2022 |
| CN | 101323643 | A | 17 December 2008 | WO | 2008154814 | A1 | 24 December 2008 |
| | | | | JP | 2010529967 | A | 02 September 2010 |
| | | | | JP | 5372917 | B2 | 18 December 2013 |
| | | | | KR | 20100009600 | A | 27 January 2010 |
| | | | | KR | 101204229 | B1 | 26 November 2012 |
| | | | | US | 2010136008 | A1 | 03 June 2010 |
| | | | | US | 8193316 | B2 | 05 June 2012 |
| | | | | RU | 2009148020 | A | 10 July 2011 |
| | | | | RU | 2433141 | C2 | 10 November 2011 |
| | | | | BRPI | 0811333 | A2 | 16 June 2015 |
| | | | | BRPI | 0811333 | B1 | 15 September 2020 |
| | | | | BRPI | 0811333 | B8 | 25 May 2021 |
| | | | | EP | 2161287 | A1 | 10 March 2010 |
| | | | | EP | 2161287 | A4 | 11 August 2010 |
| | | | | EP | 2161287 | B1 | 04 March 2015 |
| CN | 102085367 | A | 08 June 2011 | None | | | |
| CN | 102085368 | A | 08 June 2011 | None | | | |
| CN | 110522908 | A | 03 December 2019 | WO | 2019223581 | A1 | 28 November 2019 |
| | | | | TW | 202011990 | A | 01 April 2020 |
| | | | | US | 2021087253 | A1 | 25 March 2021 |
| US | 2021363223 | A1 | 25 November 2021 | TW | 202208414 | A | 01 March 2022 |
| | | | | US | 2021388054 | A1 | 16 December 2021 |
| | | | | WO | 2021226551 | A1 | 11 November 2021 |
| | | | | JP | 2023525032 | A | 14 June 2023 |
| | | | | CL | 2022003104 | A1 | 14 July 2023 |
| | | | | PE | 20230494 | A1 | 23 March 2023 |
| | | | | CO | 2022017780 | A2 | 17 March 2023 |
| | | | | WO | 2021226553 | A2 | 11 November 2021 |
| | | | | WO | 2021226553 | A3 | 28 April 2022 |
| | | | | EP | 4146684 | A2 | 15 March 2023 |
| | | | | CA | 3178882 | A1 | 11 November 2021 |
| | | | | BR | 112022022433 | A2 | 13 December 2022 |

Form PCT/ISA/210 (patent family annex) (July 2022)

**INTERNATIONAL SEARCH REPORT**
**Information on patent family members**

International application No.

**PCT/CN2023/136926**

| Patent document cited in search report | Publication date (day/month/year) | Patent family member(s) | | | Publication date (day/month/year) |
|---|---|---|---|---|---|
| | | AU | 2021267276 | A1 | 15 December 2022 |
| | | US | 2023241168 | A1 | 03 August 2023 |
| | | KR | 20230029621 | A | 03 March 2023 |
| | | IL | 297980 | A | 01 January 2023 |
| | | BR | 112022022524 | A2 | 13 December 2022 |
| | | JP | 2023525033 | A | 14 June 2023 |
| | | EP | 4146683 | A1 | 15 March 2023 |
| | | CA | 3178885 | A1 | 11 November 2021 |
| | | IL | 297981 | A | 01 January 2023 |
| | | AU | 2021268033 | A1 | 15 December 2022 |
| | | KR | 20230029622 | A | 03 March 2023 |
| | | US | 11274140 | B2 | 15 March 2022 |

Form PCT/ISA/210 (patent family annex) (July 2022)

**EP 4 527 375 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5969102 A
- US 6316222 B
- US 6500428 B
- US 569245
- US 09627206 B
- CN 101323643 A
- CN 113613675 A

### Non-patent literature cited in the description

- **ROBERT G. STRICKLEY et al.** A review of formulations of commercially available antibodies.. *Journal of Pharmaceutical Sciences*, 2021, vol. 110, 2590-2608
- **YANAN CUI et al.** Monoclonal antibodies: formulations of marketed products and recent advances in novel delivery system. *Drug Development and Industrial Pharmacy*, 2017, vol. 43 (4), 519-530
- **STEFAN R. SCHMIDT**. Production Challenges for Complex Biologics: Fusion Proteins. *American Pharmaceutical Review*, 2017, 1-5
- *J.biol.chem*, 1968, vol. 243, 3558